# EUROPÄISCHE PATENTSCHRIFT

(11) **EP 0 565 511 B1**
(45) Veröffentlichungstag und Bekanntmachung des Hinweises auf die Patenterteilung: **16.06.1999**
(21) Anmeldenummer: 93890046.1
(22) Anmeldetag: 19.03.1993
(51) Int. Cl.: C12N 9/74, C12N 9/48, C12N 11/14

(54) **Verfahren zur Spaltung von Proenzymen**
Method of cleaving proenzymes
Procédé pour cliver des proenzymes

(30) Priorität: 06.04.1992 AT 71292
(43) Veröffentlichungstag der Anmeldung: 13.10.1993
(73) Patentinhaber: IMMUNO Aktiengesellschaft, A-1221 Wien (AT)
(72) Erfinder: Turecek, Peter, Dr., A-1190 Wien (AT)
(74) Vertreter: Weinzinger, Arnulf, Dipl.-Ing.

(56) Entgegenhaltungen:
- EP-A- 0 378 798
- FEDERATION PROCEEDINGS Bd. 41, Nr. 3, 1982, US Seite 763 H. VAN KLEY ET AL. 'Alterations of activity of pancreatic proteinases by detergents'

## Beschreibung

Die Erfindung betrifft ein Verfahren zur kontrollierten, eingeschränkten proteolytischen Spaltung von Proenzymen zur Gewinnung von Enzymen, wobei das Proenzym mit einer Protease behandelt wird. Insbesondere betrifft die Erfindung ein Verfahren zur Gewinnung von Thrombin aus Prothrombin.

Ein derartiges Verfahren ist aus der EP-A-0 378 798 bekannt. Nach diesem Verfahren wird Prothrombin aus Plasma oder einer Plasmafraktion an einen festen Träger adsorbiert (immobilisiert) und das Adsorbat mit Ca²+-Ionen behandelt. Die Ca Ca²+-Ionen werden in einer Konzentration bis zu 30 mM eingesetzt und bewirken - zusammen mit ebenfalls aus dem Plasma stammenden und am Träger adsorbierten Proteasen - die Abspaltung des Thrombins vom Adsorbat. Als fester Träger werden Methacryl- und Acryl-Copolymere verwendet.

Es ist weiters bekannt, daß Trypsin gelöstes Prothrombin in Fragmente mit niedrigem Molekulargewicht abbaut, wobei ein völliger Verlust der Thrombinaktivität festzustellen ist (Biochim.Biophys.Act. 329, 221-232 (1973)); der proteolytische Abbau des Prothrombins endet somit nicht beim Thrombin, wodurch sich diese Methode nicht zur Thrombin-Gewinnung eignet.

Alle Verfahren der eingangs genannten Art führen je nach angewandter Protease und Behandlungsbedingungen zu unterschiedlichen Prothrombin-Spaltprodukten, welche zum Teil therapeutisch (z.B. Thrombin), zum Teil diagnostisch und zur Gewinnung spezifischer Antikörper verwendet werden können. Für alle diese Anwendungen ist es jedoch erforderlich, daß die Protein-Fragmente in hoher Reinheit vorliegen, was naturgemäß arbeitsaufwendig ist, da alle bekannten Spaltungsmethoden zu einer Vielzahl von Fragmenten führen. Ein weiterer Nachteil dieser vielstufigen und zeitraubenden Reinigungsverfahren besteht darin, daß sie unvermeidlich von großen Ausbeuteverlusten begleitet sind.

Bei einer Untersuchung der Effekte von Detergentien auf die Spaltung von spezifischen synthetischen Substraten durch proteolytische Enzyme, wie Trypsin, Chymotrypsin und Elastane, wurden Änderungen der Enzymaktivität von 2,5-facher Aktivierung bis zur kompletten Inhibierung gefunden (Fed.Prog.41(3), Seite 1982). In Am.J.Physical 201(2) (1961), Seiten 298-302 konnte gezeigt werden, daß die Generierung von Thrombin durch Trypsin in Anwesenheit eines Lipids (Asolectin) gesteigert wird.

Die Erfindung setzt sich zum Ziel, diese Nachteile zu beseitigen und ein verbessertes Verfahren zur Gewinnung von Enzymen aus Proenzymen zur Verfügung zu stellen, wobei sich dieses Verfahren insbesondere zur einfachen Gewinnung von Thrombin eignen soll.

Dieses Ziel wird bei dem eingangs beschriebenen Verfahren dadurch erreicht, daß die Behandlung mit der Protease in Gegenwart eines Detergens oder in Gegenwart einer chaotropen Substanz, ausgenommen gerinnungsaktive Salze, durchgeführt wird und das Detergens oder die chaotrope Substanz nach Durchführung der Behandlung mit Proteasen abgetrennt wird.

Die Erfindung beruht auf der Erkenntnis, daß die proteolytische Spaltung von Proenzymen, wie Prothrombin, gezielt erfolgt bzw. gesteuert werden kann, wenn sie in Gegenwart eines Detergens oder in Gegenwart einer chaotropen Substanz durchgeführt wird. Es hat sich gezeigt, daß das Spaltungsmuster der Proteine je nach Art und Konzentration dieser anwesenden Stoffe verschieden ist. Diese Erkenntnis eröffnet die Möglichkeit, das Reaktionsmilieu so zu wählen, daß nur wenige und ganz bestimmte Protein-Fragmente gebildet werden.

Eine bevorzugte Variante des erfindungsgemäßen Verfahrens besteht darin, daß ein Proenzym eingesetzt wird, welches an einem festen Trägermaterial, insbesondere einem schwerlöslichen Salz oder Chelat eines zweiwertigen Metalls, vorzugsweise eines Erdalkalimetalls, immobilisiert ist. Diese Variante gestattet eine erleichterte Gewinnung des vom adsorbierten Proenzym abgespaltenen Protein-Fragmentes, da der restliche Teil des Proenzyms - am Träger adsorbiert - mit dem Träger einfach von der Reaktionslösung abgetrennt werden kann.

Gemäß dem in der EP-A - 0 378 798 beschriebenen Verfahren werden als Träger Copolymere verwendet, welche jedoch den Nachteil haben, daß sie Acryl- und Methacrylmonomere in die Thrombin-hältige Lösung abgeben, die nur schwer wieder entfernt werden können und damit die herzustellende pharmazeutische Präparation verunreinigen. Ein solches Ausbluten ("leakage") ist für alle Arten von organischen polymeren Trägern bekannt. Es hat sich aber gezeigt, daß die erfindungsgemäße, gezielte Spaltung eines Proenzyms, insbesondere Prothrombins, nicht nur auf Copolymeren gelingt, sondern auch anderen Trägern, z.B. auf schwer löslichen Salzen wie Ca₃(PO₄)₂, CaSO₄, CaCO₃, BaSO₄, BaCO₃ oder Ba-Citrat. Die Salze können entweder einer Prothrombin-hältigen Lösung beigegeben oder auch durch Fällung in einer Prothrombin-hältigen Lösung erst gebildet werden.

Ein besonderer Vorteil der zweiwertigen Ionen des Trägers ist weiters, daß sie Prothrombin selektiv über ihren gamma-Carboxyterminus binden. Die Protein-Fragmente, die bei der proteolytischen Behandlung des Prothrombins entstehen, haben auf Grund des fehlenden gamma-Carboxyterminus keine Affinität zu zweiwertigen Ionen und damit zum Träger.

Als weitere Träger eignen sich auch Hydroxyapatit, ein Hydroxyapatitgel oder ein mit einem zweiwertigen Kation beladener metallchelat-affinitätschromatographischer Träger (z.B. Pharmacia Chelating Sepharose^{R}).

Zur proteolytischen Spaltung von Proenzymen eignet sich eine Vielzahl von Proteasen (z.B. Chymotrypsin, Dispase, Endopeptidase Arg-C, Endoproteinase Lys-C, Endoproteinase Glu-C, Endoproteinase Asp-N, Faktor Xa, Kallikrein, Papain, Pepsin, Plasmin, Pronase, Proteinase K, Staphylocoagulase, Subtilisin, Thrombin, Trypsin (insbesondere human, bovin, porcin), trypsinartige Proteasen aus Arthropoden oder Mikroorganismen, wie z.B. Streptomyces griseus-Trypsin, Serin-Proteasen aus Giftschlangen wie Angkistrodon rhodostoma, Bothrops atrox, Dispholidus typus, Echis carinatus, Naja nigrocollis, Oxyuranus scutellatus scutellatus, u.a.). Bevorzugt eingesetzt werden als Protease Trypsin, Chymotrypsin, Kallikrein, Dispase oder die Endoproteinasen Glu-C, Lys-C oder Asp-N. Es können auch rekombinante Proteasen eingesetzt werden.

Es hat sich gezeigt, daß ein Proenzym in Gegenwart eines Detergens so gezielt gespalten werden kann, daß das zu gewinnende Enzym den Hauptanteil des Fragmentgemisches bildet. Als Detergens eignet sich Desoxycholat (DOC), welches vorzugsweise verwendet wird, und auch andere, wie Dodecylsulfat (SDS), CHAPS, Polyoxyethylen-Derivate wie Brij, Tween, Triton und Pluronic. Die Anwesenheit des Detergens erleichert weiters die Desorption der entstandenen Protein-Fragmente vom Träger.

Typische chaotrope Substanzen, die im erfindungsgemäßen Verfahren zur Anwendung kommen, sind etwa Harnstoff, Guanidinium-Hydrochlorid sowie Thiocyanate, jedoch sind gerinnungsaktive Salze, wie z.B. Calciumsalze, die gleichfalls chaotrop wirken, nicht zweckmäßig.

Der erhaltene Überstand, der das gewünschte Enzym enthält, kann weiteren Reinigungsschritten unterworfen werden. Vorteilhaft können dafür Gelpermeationschromatographie oder Affinitätschromatographie angewandt werden. Um möglichst konzentrierte Proben zu gewinnen, empfiehlt sich der Einsatz affinitätschromatographischer Methoden. Als gut geeignet haben sich triazinaffinitätschromatographische Träger mit Liganden vom Typ des Cibacron^{R}-Blue F3GA (hergestellt von der Firma Ciba Geigy) oder Procion^{R}-Red HE-3B (hergstellt von der Firma ICI) oder verwandte Farbstoffe erwiesen. Die Protein-Fragmente können direkt aus dem Desorptionsüberstand nach erfolgter Festphasenaktivierung an die jeweilige Affinitätsmatrix (z.B Fractogel^{R} TSK AF-Blue (hergestellt von der Firma Merck), Blue-Sepharose^{R} CL-6B (hergestellt von der Firma Pharmacia)) im Batch oder in der gepackten Säule adsorbiert werden. Anschließend werden die Protein-Fragmente durch Waschen mit Puffer vom Detergens und schließlich mit einem hochmolaren (z.B. 1M) Chaotrop (z.B. KSCN oder NH₄SCN) eluiert.

Das Eluat kann über Gelpermeationschromatographie (z.B. über Sephadex G25), Diafiltration oder Dialyse vom Chaotrop befreit und in einen geeigneten Puffer oder Salzlösung gebracht werden. Die weitere Reinigung zur Homogenität kann in bekannter Weise über Reverse-Phasen-Chromatographie, Affinitätschromatographie, oder Gelpermeationschromatographie erfolgen.

Das erfindungsgemäße Verfahren eignet sich besonders gut zur Gewinnung von reinem Thrombin. Die Erfindung betrifft deshalb auch die Herstellung einer Thrombin-hältigen, pharmazeutischen Präparation, wobei eine bevorzugte Ausführungsform dadurch gekennzeichnet ist, daß
- eine Prothrombin-hältige Lösung mit einem festen Träger, insbesondere einem schwerlöslichen Salz oder Chelat eines zweiwertigen Metalls, vorzugsweise eines Erdalkalimetalls, in Kontakt gebracht wird, um Prothrombin am Träger zu immobilisieren,
- das immobilisierte Prothrombin mit einer Protease, insbesondere Trypsin, in Gegenwart eines Detergens, vorzugsweise Desoxycholat, behandelt wird, wobei eine Thrombin-hältige Lösung erhalten wird, welche abgetrennt, gereinigt und
- zu einer pharmazeutischen Präparation verarbeitet wird.

Es wurde festgestellt, daß durch die Detergensbehandlung eine deutliche Herabsetzung einer Virusaktivität erreicht wird, falls ein Ausgangsprodukt verwendet wird, welches aus einem mit Virus kontaminierten Pool stammt. Es wurde in einem Fall gefunden, daß in einer nach dem erfindungsgemäßen Verfahren hergestellten Thrombinpräparation keine Vaccinia-Viren nachweisbar waren, obwohl das Ausgangsmaterial (ein Fermentationsüberstand) Vaccinia enthalten hatte. Natürlich können im Rahmen des erfindungsgemäßen Verfahrens auch zusätzliche Maßnahmen zur Inaktivierung eventuell vorhandener infektiöser Agentien, so beispielsweise eine Dampf-Hitze-Behandlung eines lyophilisierten Produktes, vorgenommen werden.

Das erfindungsgemäße Verfahren zur Gewinnung von Thrombin wird zweckmäßigerweise folgendermaßen durchgeführt:

Zunächst wird eine Prothrombin-hältige Lösung mit dem festen Träger in Kontakt gebracht, um Prothrombin zu adsorbieren. Als Prothrombin-hältige Lösung können nicht nur Plasma oder Plasmafraktionen, sondern auch rekombinantes Prothrombin enthaltendes Zellkulturüberstandsmedium verwendet werden. Nach erfolgter Immobilisierung wird das am Träger gebundene Prothrombin aus der Lösung zweckmäßigerweise abgetrennt und gewaschen, um unspezifisch gebundene Proteine, die das spätere Endprodukt verunreinigen könnten, zu entfernen. Danach wird das immobilisierte Prothrombin in Gegenwart von Desoxycholat mit der Protease behandelt, um das Thrombin vom Prothrombin abzuspalten, wobei eine Thrombin-hältige Lösung und ein Festkörper erhalten werden, welcher Festkörper von der Lösung abgetrennt wird. Dies geschieht durch Sedimentation oder Filtration.

Der Thrombin-hältige Überstand kann den oben beschriebenen Reinigungsschritten unterworfen werden. Bevorzugt wird eine Affinitätschromatographie mit Fractogel® TSK-AF Blue (Merck) mit anschließender Sephadex-G25 Chromatographie durchgeführt.

Anschließend wird die erhaltene reine Thrombin-hältige Lösung gegebenenfalls durch Ultrazentrifugation oder Lyophilisation konzentriert und zu einer pharmazeutischen Präparation aufgearbeitet.

Eine besondere Ausführungsform der vorliegenden Erfindung liegt in einem Verfahren zur kontrollierten, eingeschränkten proteolytischen Spaltung von Plasminogen bzw. anderen Proenzymen aktivierter Blutgerinnungsfaktoren zur Gewinnung von Plasmin bzw. anderen aktivierten Blutgerinnungsfaktoren, durch Behandlung mit Proteasen, welches dadurch gekennzeichnet ist, daß die Behandlung in Gegenwart eines Detergens oder in Gegenwart einer chaotropen Substanz, ausgenommen gerinnungsaktive Salze, durchgeführt wird.

Mit den folgenden Beispielen wird die Erfindung noch näher erläutert:

### Bereitung des immobilisierten Prothrombins

Zellkulturüberstandsmedium gemäß der PCT-Anmeldung WO 91/11519, welches rekombinantes humanes Prothrombin enthält, wird mit 5 g pulverisiertem Ca₃(PO₄)₂ pro 100 IE Prothrombin versetzt und bei 4°C eine Stunde leicht gerührt. Danach wird die feste Phase bei 5000 g abzentrifugiert, das erhaltene Pellet in 40 ml 20 mM Tris/HCl-Puffer (pH 7,4) resuspendiert, 10 min gerührt und bei 5000 g erneut abzentrifugiert. Dieser Vorgang wird mit 40 ml 5 % (G/V) Ammoniumsulfat im genannten Puffer und zuletzt wieder mit 40 ml reinem Puffer wiederholt.

In gleicher Weise kann als Prothrombin-hältiges Ausgangsmaterial z.B. ein partieller Prothrombinkomplex, also eine Mischung der Faktoren II, IX und X, zur Bereitung des immobilisierten Prothrombins eingesetzt werden.

### Beispiel 1: Einfluß des Reaktionsmilieus auf die tryptische Spaltung von immobilisiertem Prothrombin

Um den Einfluß von chaotropen Substanzen und Detergentien auf die tryptische Spaltung von Prothrombin zu
dokumentieren, wurden zunächst vier Lösungen (A - D) eines Tris/HCl-Puffers (20 mM; pH 8,3) mit den folgenden Zusätzen bereitet (die vierte Puffer-Lösung (D) diente als Vergleich):
Lösung A: Harnstoff (0,5 M)
Lösung B: Na-Desoxycholat (0,05 M)
Lösung C: Na-Dodecylsulfat (0,05 M)
Lösung D: kein Zusatz

1 ml der Lösungen A - D wurden anschließend je 200 mg pufferfeuchtes, gewaschenes Pellet (wie oben beschrieben hergestellt) zugegeben und mit 20 µl eines 20 mM Tris/HCl-Puffers, enthaltend 1 mg Trypsin/ml, bei Raumtemperatur geschüttelt. Nach 1, 2 und 3 Stunden wurden jeweils Aliquote der Suspensionen gezogen, die feste Phase abzentrifugiert und die Überstände mittels Immun-Western-Blot auf die jeweilige Fragmentzusammensetzung untersucht.

In Tabelle 1 sind die Peakflächenintegrale der Fragmentbanden bei 12, 19, 23, 25,5, 33, 35 und 44 kD nach densitometrischer Immun-Western-Blot-Messung eingetragen. Es ist ersichtlich, daß sich in Abhängigkeit des gewählten Reaktionsmilieus verschieden große tryptische Fragmente in unterschiedlichen Mengen nachweisen lassen. Im Gegensatz zur 3-stündigen Inkubation in reinem Tris-Puffer (Lösung D) bleiben in allen anderen Lösungen (A - C) Fragmente im Molekülmassebereich über 30 kD erhalten. Die Fragmente bei 33 kD und bei 35 kD entsprechen Thrombin, wobei 33 kD der aktiven Form zuzuordnen ist.

In Gegenwart von Desoxycholat (Lösung B) wird aktives Thrombin als Hauptfragment gebildet, welches auch nach 3-stündiger Trypsin-Behandlung nicht zu kleineren Oligopeptiden abgebaut wird.

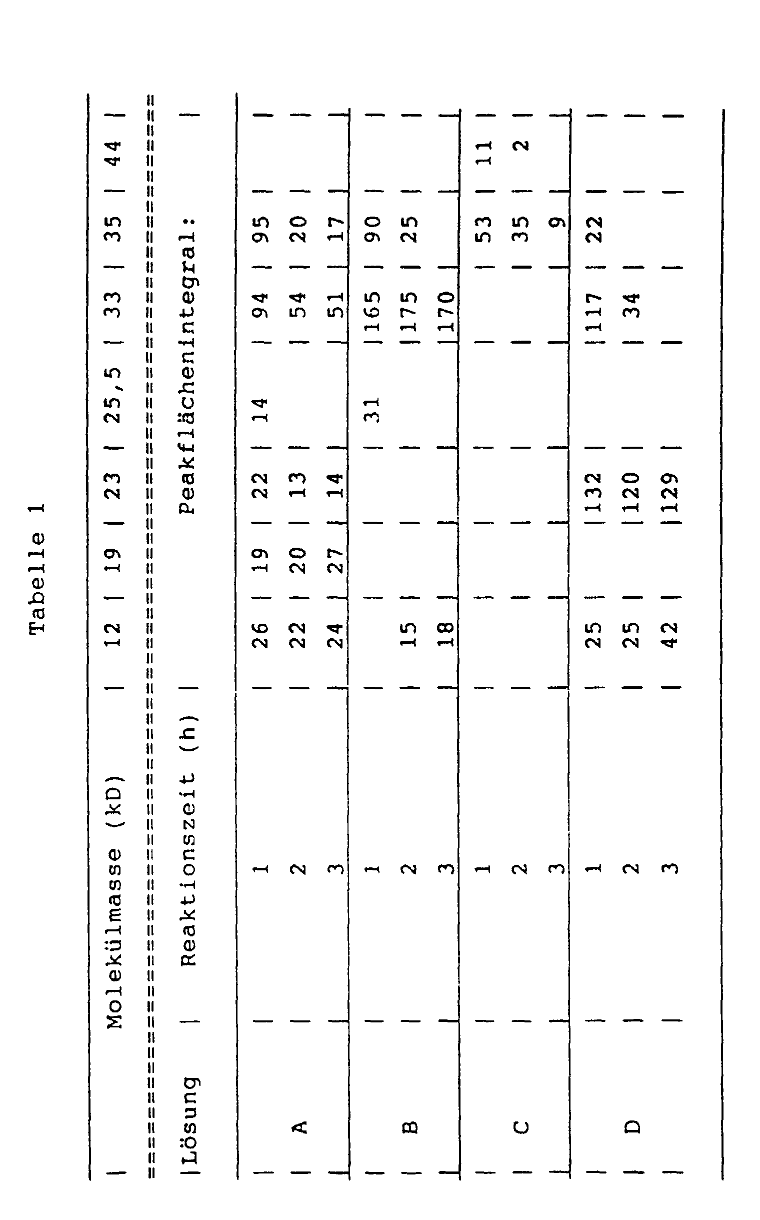

### Beispiel 2: Spaltung von immobilisiertem Prothrombin mittels verschiedener Proteasen (in Gegenwart eines Detergens)

Aus einem Prothrombin enthaltenden Zellkulturüberstandsmedium wurde, wie oben beschrieben, Prothrombin an Ca₃(PO₄)₂ adsorbiert und gewaschen.

8 Proben zu je 250 mg pufferfeuchtes Adsorbat wurden in 1 ml 20 mM Tris/HCl-Puffer, pH 8,3, der 200 mM an Na-Desoxycholat enthielt, suspendiert und mit 50 µl der folgenden acht Enzymlösungen versetzt:
* Kallikrein aus Schweinepankreas, 250 U/ml in 20 mM TBS, pH 8,3
* Dispase 1 aus Bacillus polymyxa, 1 mg/ml in 20 mM TBS, pH 8,3
* α-Chymotrypsin aus Rinderpankreas, 350 U/ml in 20 mM TBS, pH 8,3
* Trypsin aus Schweinepankreas, 0,76 mg/ml in 20 mM TBS, pH 8,3
* Endoproteinase Glu-C aus Staphylococcus aureus V8, 1 mg/ml in 20 mM TBS, pH 8,3
* Endoproteinase Lys-C aus Lysobacter enzymogenes, 0,1 mg/ml in 20 ITIM TBS, pH 8,3
* Endoproteinase Asp-N aus Pseudomonas fragi, 0,04 mg/ml in 20 mM TBS, pH 8,3
* Faktor Xa, human, 20 U/ml

Die Bezeichnung 20 mM TB5 (TBS = tris buffered saline) bezieht sich auf einen 20 mM Tris-HCl-Puffer (pH 8,3), welcher 0,9 % NaCl enthält.

Die Ansätze wurden zwei Stunden, im Falle des Kallikrein 20 Stunden, bei Raumtemperatur unter Schütteln inkubiert. Danach wurden Aliquote der Ansätze mit SDS-Probenpuffer versetzt (1 : 1) und mittels Immun-Western-Blot auf ihre Fragmentzusammensetzung untersucht.

Die Peakflächenintegrale der Fragmentbanden bei 12, 18, 19, 20, 23, 33, 34, 35, 44, 47, 50, 52, 54, 55, 71 und 75 kD nach densitometrischer Immun-Western-Blot-Messung sind in Tabelle 2 wiedergegeben.

Es ist ersichtlich, daß sich mit den genannten Proteasen eine Reihe von Fragmenten im Molekülmassenbereich von 12 bis 71 kD darstellen lassen. Das dem aktiven Thrombin entsprechende Fragment (33 kD) läßt sich mittels tryptischem Abbau von Prothrombin in besonders hoher Ausbeute gewinnen.

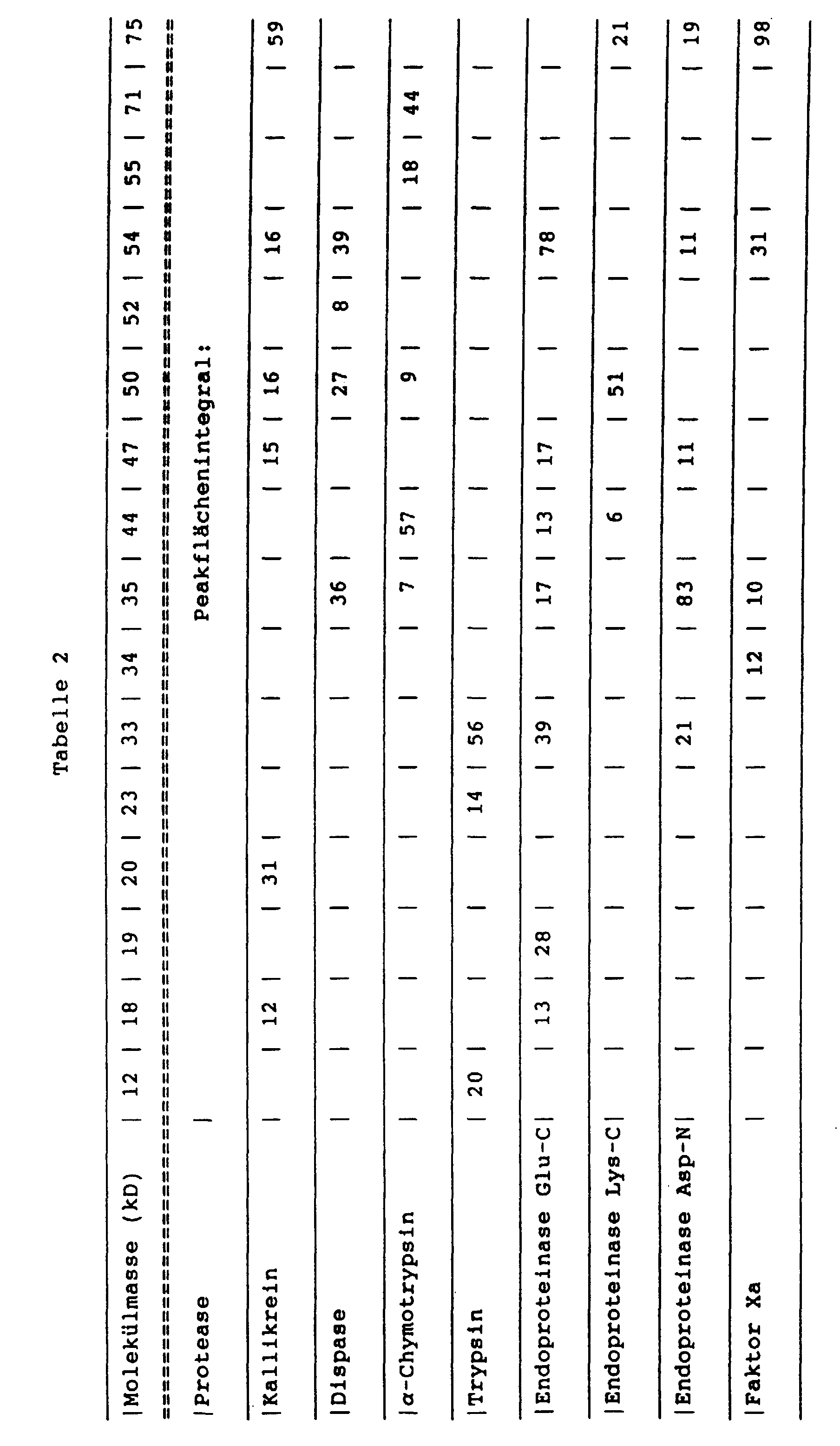

### Beispiel 3: Spaltung von immobilisiertem Prothrombin in Gegenwart von Desoxycholat in verschiedenen Konzentrationen

Wie oben beschrieben, wurde zunächst rekombinantes Prothrombin aus einem Fermentationsüberstand an Ca₃(PO₄)₂ adsorbiert.

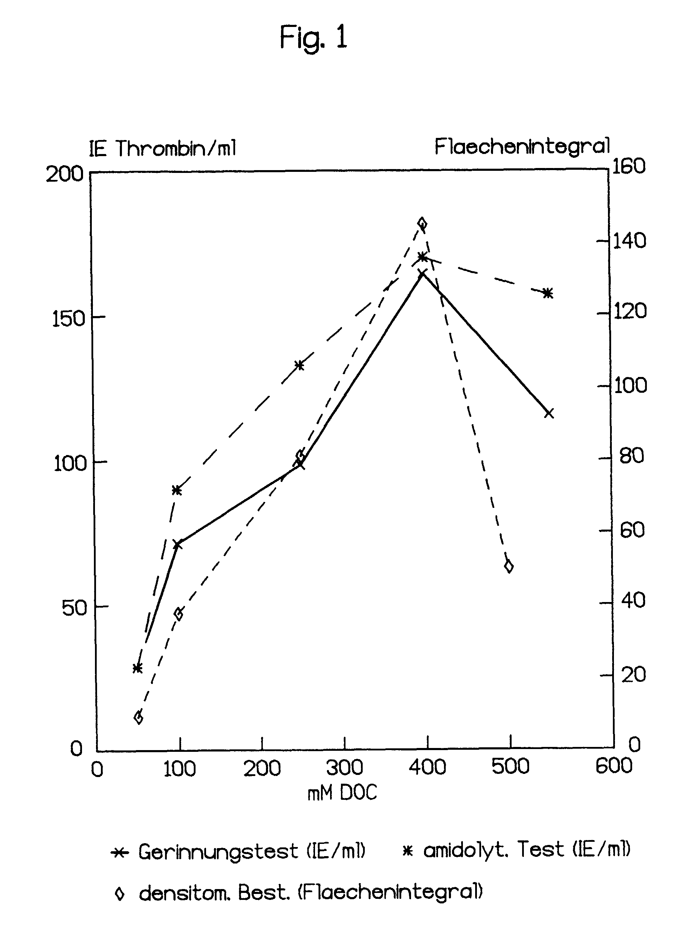

5 Proben zu je 0,6 g feuchtes Adsorbat wurden mit je 3 ml 20 mM Tris/HCl-Puffer, pH 8,3, der Na-Desoxycholat in den Konzentrationen 50 mM, 100 mM, 250 mM, 350 mM und 500 mM enthielt, nach Zusatz von 60 µl einer Lösung von 0,76 mg/ml Trypsin/ml unter Schütteln 3 Stunden bei Raumtemperatur inkubiert. Danach wurden die gegen 0,9 % NaCl umgepufferten Proben auf ihre Thrombinaktivität untersucht. Dabei wurde die Thrombinzeit im Gerinnungstest mit Normalplasma sowie die amidolytische Aktivität mit dem chromogenen Substrat TH-1 (2 AcOH.H-D-CHG-Ala-Arg-p-Nitroanilid) jeweils photometrisch gegen einen Internationalen Thrombin-Standard bestimmt (Fig. 1). Die Fig. 1 zeigt, daß die Ausbeute an aktivem Thrombin (33 kD-Fragment) bei 350 mM Desoxycholat ein Maximum aufweist.

Weiters wurde festgestellt, daß das gebildete Thrombin trotz der Anwesenheit des Trypsins über einen Zeitraum von mindestens 20 Stunden nicht weiter abgebaut wird.

### Reinigung von Protein-Fragmenten des gespaltenen Prothrombins

1,5 ml einer Prothrombin-Fragmente enthaltenden, aus Beispiel 3 stammenden Lösung (350 mM DOC) wurden mit 0,1 % Trifluoressigsäure in H₂O (Laufmittel A) an eine Reverse-Phasen-HPLC-Säule (Nucleosil 100-5C18, 125 x 4 mm) adsorbiert (Flußrate: 1,7 ml/min). Danach wurde mit 0,1 % Trifluoressigsäure in Acetonitril (Laufmittel B) mit einem linearen Gradienten von 30 bis 70 % B in 30 min bei einer Flußrate von 1,7 ml/min eluiert. Durch Detektion bei 220 nm konnten 6 Haupt-Peaks (Retentionszeiten: 10,01 min; 11,96 min; 12,68 min; 13,47 min; 13,94 min; 14,47 min) identifizert werden; sie wurden getrennt gesammelt und lyophilisiert. Die weitere Analyse der getrennten Fragmente erfolgte über SDS-PAGE mit Detektion durch Coomassie-Färbung, sowie mittels Immun-Western-Blot mit einem polyklonalen Kaninchen-anti-Humanprothrombin-Antiserum. Die Molekülmassen der Fragmente wurden für die sechs Haupt-Peaks mit 9 kD, 16 kD, 21 kD, 23 kD, 12 kD und mit 33 kD bestimmt.

### Beispiel 4: Gewinnung von Thrombin

Ein pufferfeuchtes Pellet (ca. 10 g) wurde mit 50 ml einer Lösung von 0,76 mg porcinem Trypsin (Sigma T-0134) pro ml in 20 mM Tris/HCl-Puffer (pH 8,3), der 200 mM Na-Desoxycholat enthielt, 1 Stunde bei Raumtemperatur unter leichtem Rühren inkubiert. Danach wurde das Ca-Phosphat durch Zentrifugation abgetrennt. Der Überstand enthielt in erster Linie Thrombin, neben einigen wenigen anderen Fragmenten von Prothrombin.

Zur Reinigung des Überstandes wurde eine Säule mit 8 cm² Querschnittsfläche mit Fractogel^{R} TSK-AF Blue (Merck) in einer Höhe von 12 mm (Gelvolumen ≈ 9,6 ml) in 20 mM Tris/HCl-Puffer (pH 8,3) gepackt und mit demselben Puffer gewaschen. Dieser und alle nachfolgenden Schritte erfolgten bei 4°C.

Der Thrombin enthaltende Überstand (ca. 50 ml) wurde zur Adsorption mit einer Flußrate von 2 ml/min über das Gel gepumpt. Danach wurden mit 20 ml 0,5 M NaCl-Lösung, 40 ml 1,0 M NaCl-Lösung und 20 ml 20 mM Tris/HCl-Puffer (pH 7,4) bei einer Flußrate von 6 ml/min unspezifisch gebundene Prothrombinfragmente eluiert. Bei umgekehrter Flußrichtung mit 1 ml/min wurden nun Prothrombin-Fragmente mit 20 mM Tris/HCl-Puffer, der 1 M KSCN enthielt, eluiert. Das Eluat wurde über eine Durchflußzelle bei 280 nm photometrisch gemessen, insgesamt wurden 15 ml gesammelt.

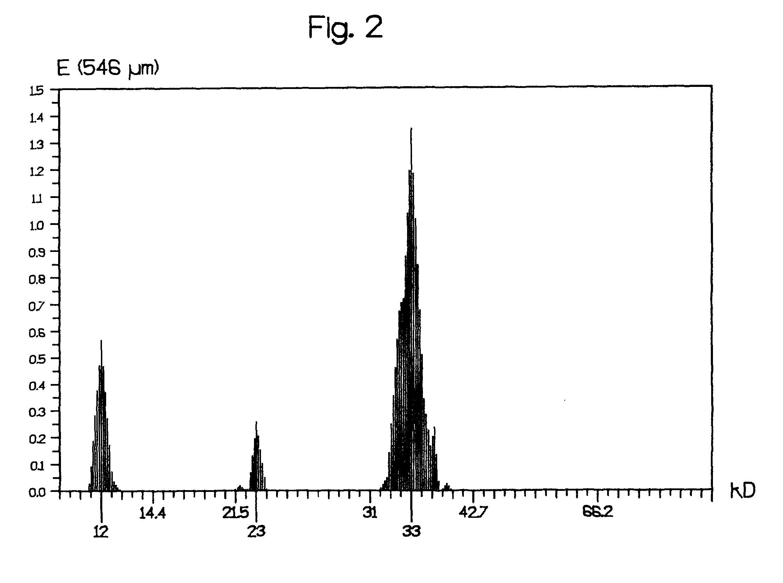

Der Proteingehalt des Eluates betrug nach Bradford 188 mg/ml. Fig. 2 zeigt die Fragmentzusammensetzung (densitometrischer Immun-Western-Blot-Scan) mit dominantem 33 kD-Fragment (aktives Thrombin). Auf Thrombin entfallen ca. 30 %, bezogen auf den Proteingehalt. Über Bestimmung der Thrombinzeit konnte für das Thrombin eine spezifische Aktivität von ca. 2200 IE/mg Protein ermittelt werden. Durch tryptische Festphasenaktivierung konnten somit aus 1 IE Prothrombin ca. 100 IE Thrombin gewonnen werden.

Die erhaltene Thrombin-hältige Lösung kann auf bekannte Weise weiter gereinigt, aufkonzentriert und zu einer pharmazeutischen Präparation weiterverarbeitet werden.

### Beispiel 5: Spaltung von gelöstem Prothrombin

Zwei Proben zu je 1 ml Prothrombin enthaltender Fermentationsüberstand wurden mit je 1 ml eines 40 mM Tris/HCl-Puffers, pH 8,3, der 0,1 M Na-Desoxycholat bzw. 0,1 M Na-Dodecylsulfat enthielt, versetzt, so daß die Detergenskonzentration in der Lösung 0,05 M betrug. Als Vergleich wurde eine dritte Probe zu 1 ml Prothrombinhältigem Fermentationsüberstand mit 40 mM Tris/HCl-Puffer ohne Detergens versetzt. Nach Zusatz von jeweils 15 ml einer Lösung von 1 mg Trypsin/ml in 20 mM Tris/HCl-Puffer, pH 8,3, der 0,9 % NaCl enthielt, wurde unter Schütteln 4 Stunden bei Raumtemperatur inkubiert.

Nach 1, 2, 3 und 4 Stunden wurde je ein Aliquot der Proben von 50 µl mit Laemmli-Puffer verdünnt (1 : 1), aufgekocht und elektrophoretisch analysiert. Der Immun-Western-Blot eines 12 % SDS-Polyacrylamidgels (1. Antikörper: Anti-human-FaktorII-Kaninchen-Serum; 2. Antikörper: Ziege-anti-Kaninchen-IgG-Peroxidase-Konjugat; Entwicklung mittels 4-Chlor-1-naphthol) wurde densitometrisch im Auflicht zur Quantifizierung der Fragmente untersucht. Die Ergebnisse sind in Tabelle 3 eingetragen.

**Tabelle 3**

| Molekülmasse | (kD) | 19 | 25,5 | 33 | 35 |
|---|---|---|---|---|---|
| Zusatz | Reaktionszeit (h) | | Peakflächenintegral | | |
| | 1 | | | 10 | |
| Desoxycholat | 2 | | | 9 | |
| | 3 | | | 7 | |
| | 4 | | | 6 | |
| Dodecylsullfat | 1 | 8 | | | 68 |
| | 2 | 6 | | | 67 |
| | 3 | 5 | | | 69 |
| | 4 | 4 | | | 67 |
| | 1 | | 4 | 9 | 4 |
| | 2 | | 3 | 1 | 0 |
| Leerwert | 3 | | 0 | 0 | 0 |

Obwohl das erfindungsgemäße Verfahren im einzelnen anhand der Gewinnung von Thrombin aus Prothrombin beschrieben worden ist, ist es für den Fachmann verständlich, daß ähnliche Spaltungen anderer Proenzyme als Prothrombin in analoger Weise durchgeführt werden können; beispielsweise können auf diese Art Plasmin aus Plasminogen bzw. aktivierte Blutgerinnungsfaktoren aus ihren Proenzymen gewonnen werden.

## Patentansprüche

1. Verfahren zur kontrollierten, eingeschränkten proteolytischen Spaltung von Proenzymen zur Gewinnung von Enzymen durch Behandlung mit Proteasen, dadurch gekennzeichnet, daß die Behandlung in Gegenwart eines Detergens oder in Gegenwart einer chaotropen Substanz, ausgenommen gerinnungsaktive Salze, durchgeführt wird und das Detergens oder die chaotrope Substanz nach Durchführung der Behandlung mit Proteasen abgetrennt wird.

2. Verfahren nach Anspruch 1, dadurch gekennzeichnet, daß ein Proenzym eingesetzt wird, welches an einem festen Trägermaterial, insbesondere einem schwerlöslichen Salz oder Chelat eines zweiwertigen Metalls, vorzugsweise eines Erdalkalimetalls, immobilisiert ist.

3. Verfahren nach einem der Ansprüche 1 oder 2, dadurch gekennzeichnet, daß als Protease Trypsin, Chymotrypsin, Kallikrein, Dispase oder die Endoproteinasen Glu-C, Lys-C oder Asp-N eingesetzt wird.

4. Anwendung des Verfahrens nach den Ansprüchen 1 bis 3 zur Gewinnung von Thrombin aus Prothrombin.

5. Verfahren zur Herstellung einer Thrombin-hältigen pharmazeutischen Präparation, gekennzeichnet durch die Kombination der Maßnahmen, daß
- eine Prothrombin-hältige Lösung mit einem festen Träger, insbesondere einem schwerlöslichen Salz oder Chelat eines zweiwertigen Metalls, vorzugsweise eines Erdalkalimetalls, in Kontakt gebracht wird, um Prothrombin am Träger zu immobilisieren,
- das immobilisierte Prothrombin mit einer Protease, insbesondere Trypsin, in Gegenwart eines Detergens, vorzugsweise Desoxycholat, behandelt wird, wobei eine Thrombin-hältige Lösung erhalten wird, welche abgetrennt, gereinigt und
- zu einer pharmazeutischen Präparation verarbeitet wird.

6. Verfahren zur kontrollierten, eingeschränkten proteolytischen Spaltung von Plasminogen bzw. anderen Proenzymen aktivierter Blutgerinnungsfaktoren zur Gewinnung von Plasmin bzw. anderen aktivierten Blutgerinnungsfaktoren, durch Behandlung mit Proteasen, dadurch gekennzeichnet, daß die Behandlung in Gegenwart eines Detergens oder in Gegenwart einer chaotropen Substanz, ausgenommen gerinnungsaktive Salze, durchgeführt wird.

## Claims

1. A method for the controlled, limited proteolytic cleavage of proenzymes, for recovering enzymes by treatment with proteases, characterised in that the treatment is effected in the presence of a detergent or in the presence of a chaotropic substance, with the exception of coagulatively active salts, and the detergent or the chaotropic substance is separated after carrying out the treatment with proteases.

2. A method according to claim 1, characterised in that a proenzyme is used which has been immobilized on a solid carrier material, in particular on a hardly soluble salt or chelate of a bivalent metal, preferably of an alkaline earth metal.

3. A method according to any one of claims 1 or 2, characterised in that trypsin, chymotrypsin, kallikrein, dispase or the endoproteinases Glu-C, Lys-C or Asp-N is used as protease.

4. Use of the method according to claims 1 to 3 for recovering thrombin from prothrombin.

5. A method of preparing a thrombin-containing pharmaceutical preparation, characterised by the combination of the measures that
- a prothrombin-containing solution is contacted with a solid carrier, in particular a hardly soluble salt or a chelate of a bivalent metal, preferably an alkaline earth metal, so as to immobilize prothrombin on the carrier,
- the immobilized prothrombin is treated with a protease, in particular trypsin, in the presence of a detergent, preferably deoxycholate, wherein a thrombin-containing solution is obtained which is separated, purified and
- processed into a pharmaceutical preparation.

6. A method for the controlled, limited proteolytic cleavage of plasminogen or other protenzymes of activated blood coagulation factors for recovering plasmin or other activated blood coagulation factors, respectively, by treatment with proteases, characterised in that the treatment is carried out in the presence of a detergent or in the presence of a chaotropic substance, with the exception of coagulatively active salts.

## Revendications

1. Procédé de clivage protéolytique limité, contrôlé, de proenzymes pour l'obtention d'enzymes par traitement avec des protéases, caractérisé en ce que le traitement est réalisé en présence d'un détergent ou en présence d'une substance chaotrope, à l'exception des sels actifs pour la coagulation, et le détergent où la substance chaotrope est séparé après la réalisation du traitement avec des protéases.

2. Procédé selon la revendication 1, caractérisé en ce que l'on utilise une proenzyme qui est immobilisée sur un matériau support solide, en particulier un sel ou chélate peu soluble d'un métal divalent, de préférence un métal alcalino-terreux.

3. Procédé selon l'une des revendications 1 et 2, caractérisé en ce que l'on utilise comme protéase la trypsine, la chymotrypsine, la kallicréine, la dispase ou les endoprotéinases Glu-C, Lys-C ou Asp-N.

4. Utilisation du procédé selon les revendications 1 à 3 pour l'obtention de la thrombine à partir de la prothrombine.

5. Procédé de production d'une préparation pharmaceutique contenant de la thrombine, caractérisé par la combinaison des mesures selon lesquelles
- une solution contenant de la prothrombine est mise en contact avec un support solide, en particulier un sel ou chélate peu soluble d'un métal divalent, de préférence d'un métal alcalino-terreux, pour immobiliser la prothrombine sur le support,
- la prothrombine immobilisée est traitée avec une protéase, en particulier la trypsine, en présence d'un détergent, de préférence le désoxycholate, de sorte que l'on obtient une solution contenant de la thrombine qui est séparée, purifiée et
- transformée en une préparation pharmaceutique.

6. Procédé de clivage protéolytique limité, contrôlé, du plasminogène ou d'autres proenzymes de facteurs de coagulation du sang activés pour l'obtention de la plasmine ou d'autres facteurs de coagulation du sang activés par traitement avec des protéases, caractérisé en ce que le traitement est réalisé en présence d'un détergent ou en présence d'une substance chaotrope, à l'exception des sels actifs pour la coagulation.
